Europäisches Patentamt

European Patent Office

Office européen des brevets

(11) Publication number: **0 101 298**
**B1**

(12) **EUROPEAN PATENT SPECIFICATION**

(45) Date of publication of patent specification: **13.01.88**

(21) Application number: **83304625.3**

(22) Date of filing: **10.08.83**

(51) Int. Cl.⁴: **A 61 F 13/02,** A 61 F 15/00

(54) **Easy opening packaged bandage.**

(30) Priority: **11.08.82 US 407029**

(43) Date of publication of application:
**22.02.84 Bulletin 84/08**

(45) Publication of the grant of the patent:
**13.01.88 Bulletin 88/02**

(84) Designated Contracting States:
**AT BE CH DE FR GB IT LI NL SE**

(56) References cited:
**US-A-2 969 144**
**US-A-2 969 145**
**US-A-4 264 008**

(73) Proprietor: **Johnson & Johnson Products Inc.**
**501 George Street**
**New Brunswick New Jersey 08903 (US)**

(72) Inventor: **Swick, Paul Bernhard**
**7, Petunia Drive Apt. 2-6**
**North Brunswick New Jersey 08902 (US)**

(74) Representative: **Jones, Alan John et al**
**CARPMAELS & RANSFORD 43 Bloomsbury**
**Square**
**London, WC1A 2RA (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

## Description

This invention relates to adhesive bandages of the kind having a generally flexible backing coated on one surface with a pressure sensitive adhesive composition. In particular, this invention relates to packaged adhesive bandages and methods for manufacturing the same whereby the bandage may be easily and efficiently presented to the user in a ready-to-apply condition.

Adhesive bandages are now in common use for both professional and home first aid purposes, and take various shapes such as strips, patches, spots or the like in varying sizes for applying to the different parts of the body. Most such bandages in common use are packaged by first protecting the adhesive coated surface with relatively stiff facing sheets, generally co-extensive with the adhesive coating, and then overwrapping the faced bandage in a wrapper. In use, the wrapper is removed, utilizing a tear strip or tear string, and then the facing sheets are removed to expose the adhesively coated surface for applying the bandage to the wound. Such packaged bandages have received wide acceptance in that the bandage is maintained in a clean, hygienic state and can be rapidly manufactured in great quantities thereby allowing such bandages to be sold at relatively low cost.

Unfortunately, several drawbacks exist with respect to such packaged bandages. Firstly, the removal of the outer wrapper by utilizing tear strips or strings is a somewhat awkward process for the user and occasionally, either because of poor execution or manufacturing error, such process will not be successfully performed. Further, the necessity for subsequent removal of the facing sheets disdvantageously adds to the time required to apply the bandages. Still further, the removal of the facing sheets and the concomitant manipulation of the thin flexible bandage, with its now exposed tacky adhesive surface, can lead to the bandage folding over and adhering to itself prior to application to the wound and necessitate unfolding or even discarding the so-folded bandage in favour of a new one.

Several attempts have been suggested for alleviating the above-described drawbacks. Such suggestions are exemplified in US—A—2,969,144, issued to Eli A. Zackheim on January 24, 1961 and US—A—2,969,145, issued to G. Hannauer, Jr., on the same day. Basically, both these patents suggest that the problem of the facing sheets may be obviated by simply doing away with them. Instead, the adhesive bandage is folded over onto itself so as to provide a folded bandage presenting adhesive coating on both external major surfaces. The folded bandage is sandwiched between two sheets of wrapper material, each of which adheres to the adhesive coating on each external major surface of the folded bandage. Each of the wrapper sheets have portions which extend beyond the periphery of the folded bandage. These extended peripheral portions of the wrapper sheets are then peelably sealed together to enclose the bandage on all sides. In use, the two wrapper sheets are peeled apart, with the aid of tab ends, and present the bandage ready for use.

This prior suggestion does indeed obviate the user problem with respect to removal of the facing sheets and presents the bandage in a way that tends to lessen the chance of the bandage folding over and adhering to itself.

Unfortunately, this packaging method has not been commercially acceptable in that a substantial problem has been presented to the manufacturer preventing its implementation. Specifically, it has been discovered that there is no satisfactory way to cut the individual adhesive coated bandages into the various sizes and shapes without the use of facing sheets or to fold and insert the bandage within the wrapper without the facing sheets. The method utilized prior to the aforementioned suggestions, to produce bandages of the required size and shape, is to first coat a continuous web of backing material with pressure sensitive adhesive and overlie the adhesive surface with the facing sheets to form a faced continuous web. The bandages are then die cut from the faced continuous web into the desired shape. Clearly, without the facing sheets, great difficulty is encountered in die cutting the backing web in that the adhesive coated surface will contact the cutting die.

Further, the handling of the cut individual bandages for the purpose of folding and packaging them between wrapper sheets has presented greater processing difficulties in that these cut bandages lack any rigidity and cannot be processed at the high speed required for economically prudent production.

For the above reasons, there is a need for a packaged adhesive bandage system which cures the user problems that prior art solutions were directed toward curing but does so without the concomitant drawbacks associated with these prior art suggestions.

In accordance with this invention, a packaged adhesive bandage system is provided which obviates the user problems relating to facing sheets and wrapper as addressed by the prior art without concomitant processing problems. Specifically, the packaged bandage system of this invention is designed to maintain the use of facing sheets and hence retain their value as processing aids in the manufacture of packaged adhesive bandages while, at the same time, avoids user difficulties associated with the opening and applying of the bandage.

The packaged adhesive bandage of this invention comprises a generally planar backing sheet having a pressure sensitive adhesive layer coated onto one major surface thereof and a pad for absorbing body exudate located on the adhesively coated surface of the backing sheet. The bandage is folded about a transverse fold line to bring portions of the non-coated major surface into face-to-face contact so that the folded bandage presents pressure sensitive adhesive on both of its external major surfaces.

The folded bandage is sandwiched between facing sheets each having first and second major surfaces with the first major surface of each facing sheet in face-to-face contact and adhered to the pressure sensitive adhesive of the folded bandage to form a faced folded bandage. The faced folded bandage is sandwiched between wrapping sheets having first and second major surfaces, with the first major surface of each wrapping sheet in face-to-face contact with the second major surface of the facing sheet. The wrapping sheets are larger in area than the planar faced folded bandage i.e. the wrapper sheets have peripheral portions which extend on all sides beyond the sandwiched faced folded bandage and are in face-to-face relationship with each other. These peripheral portions are peelably sealed together to form a sealed, wrapped package.

In accordance with this invention, sealing means are provided for adhering the wrapping sheets to the facing sheets in the area of their face-to-face contact. Such sealing means are chosen so that the facing sheets will cling more tenaciously to the wrapper than to the pressure sensitive adhesive.

Accordingly, when the package is to be opened, the user peels apart the wrapper sheets, preferably by gripping the sheets by unsealed tab ends provided thereon. The peripheral portions of the wrapping sheets are thereby separated. Because of the prescribed tenacity of the sealing means between the wrapper sheets and the facing sheets, the same motion applied to peel apart the wrapping sheets will simultaneously peel the facing sheets from the adhesive coating of the bandage and thereby present the bandage ready for use. The facing sheets remain sealed to the wrapping sheets and may be discarded together.

It can be seen therefore that the advantages suggested in the art with respect to a peelably opened adhesive bandage package are still maintained in that the awkwardness of tear strips or strings has been avoided. Further, the need for the additional and disadvantageous step of subsequently removing facing sheets from the unwrapped bandages has also been avoided and the bandage is presented in a ready to use condition. These advantages notwithstanding, the use of facing sheets is still maintained and all of the advantages of their use as processing aids in cutting and folding the bandage remain.

A wide variety of sealing means may be chosen for sealing the wrapping sheets to the facing sheets provided, of course, that, in accordance with the teachings of this invention, such sealing means will allow the facing sheets to cling more tenaciously to the wrapping sheets than to the folded bandage when the wrapping sheets are peeled apart. In a preferred embodiment of this invention, however, the first major surface of each wrapping sheet is coated with a cohesive coating in the area in which it makes face-to-face contact with the second major surface of the facing sheet. By the term "cohesive coating" it is meant a coating which has the property of adhering to itself or to a similar coating but not to other surfaces. The second major surface of the facing sheets are likewise coated with such cohesive coating and the two cohesive coatings cooperate to form the herein prescribed sealing means.

It is preferred that irrespective of the choice of coating applied to the first major surfaces of the wrapping sheets to form the sealing means, such coating be overall applied and thereby extend onto the peripheral portions of the wrapping sheet and serve to peelably seal these portions together to enclose the bandage.

Thus, the cohesive coating described above could be employed for the purpose of both providing the sealing means with respect to the facing sheets and providing the means for peelably sealing the package.

The invention will be best understood by consideration of the following description, in combination with the drawings, wherein:

Fig. 1 is a planar view of a first embodiment of the packaged bandage of this invention, with parts removed;

Fig. 2 is a perspective view of the opened, packaged bandage of Fig. 1;

Fig. 3 is a longitudinal, cross-sectional view of the packaged bandage of Fig. 1, taken through line 3—3 of Fig. 1;

Fig. 4 is an enlargement of a portion of the packaged bandage as viewed in the circled area of Fig. 3;

Fig. 5 is an enlargement of a second portion of the packaged bandage as viewed in the circled area of Fig. 3;

Fig. 6 is a planar view of a second embodiment of the packaged bandage of this invention, with parts removed;

Fig. 7 is a perspective view of the opened packaged bandage of Fig. 6;

Fig. 8 is a longitudinal, cross-sectional view of the packaged bandage of Fig. 6, taken through line 8—8 of Fig. 6;

Fig. 9 is a transverse, cross-sectional view of the bandage of Fig. 6, taken through line 9—9 of Fig. 6;

Fig. 10 is an enlargement of a portion of the packaged bandage as viewed in the circled area of Fig. 8; and

Fig. 11 is an enlargement of a second portion of the packaged bandage as viewed in the circled area of Fig. 8.

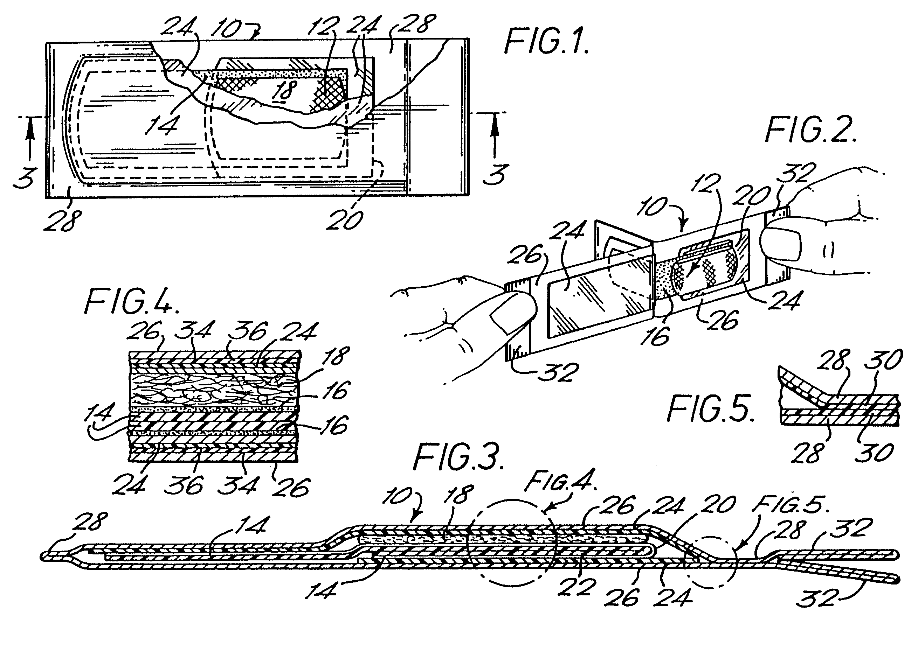

Referring to Figs. 1 and 3, illustrated therein is a first embodiment of the packaged folded bandage 10 of this invention. The folded bandage 12 comprises a generally planar flexible backing 14 which may be provided as either a woven or non woven fibrous material such as cloth, tricot mesh, paper or other fibrous cellulosic nonwoven material. Alternatively, the backing may be a film of polymeric material such as polyvinyl chloride film and may be provided with ventilation holes.

Deposited onto the backing surface 14 is a layer of pressure sensitive adhesive 16 for adhering the bandage to the body of the wearer. Pressure sensitive adhesives suitable for this purpose are well known in the art and usually comprise a mixture of natural and synthetic rubbers in varying proportions with the addition of resins or other suitable tackifiers.

Approximately centrally located on the adhesively coated surface of the backing is a pad 18 for absorbing body exudate which pad may comprise any suitable absorbent material, for instance a folded woven fabric such as gauze.

The bandage is folded about a fold line 20 so as to bring portions of the non-coated surface of the backing into face-to-face relationship along interface 22 and form the folded bandage 12 which presents the pressure sensitive adhesive 16 on each of its external major surfaces.

The folded bandage 12 is sandwiched between two facing sheets 24 to present a faced folded bandage. The facing sheets 24 which comprise relative stiff material, as compared to that of the backing, are affixed to the folded bandage by adherence with the pressure sensitive adhesive 16.

The faced folded bandage is sandwiched between wrapping sheets 26 which are generally planar and have peripheral portions 28 extending beyond the area of the faced folded bandage. Such wrapping sheets may comprise materials well known in the art for wrapping adhesive bandages and protecting the bandage prior to use. Preferably the wrapping sheets are selected to be amenable to processes for sterilizing the bandage. Typically, the wrapping sheets are comprised of lightweight, medical grade, Kraft paper or glassine.

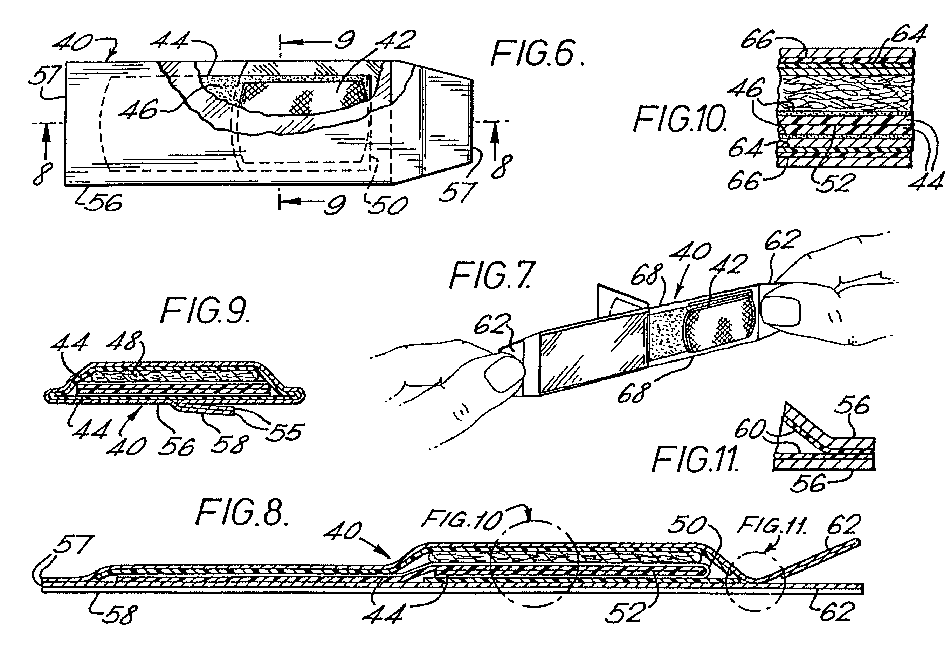

As is best illustrated in Fig. 5, the peripheral portions 28 of the wrapping sheets are peelably sealed together to enclose the faced folded bandage. This may be accomplished by coating the peripheral areas of the wrapper sheets, in the area of their face-to-face-contact, with an adhesive or cohesive layer 30. Alternatively, layer 30 may comprise a heat sealable material, capable of forming a peelable seal upon the application of heat and/or pressure. Such adhesive; cohesive and heat sealable layers are all now well known in the art. Tabs 32 are provided on at least one end of the packaged bandage to facilitate opening the package by peeling apart the peripheral portions of the wrapper sheets. These tabs may be formed by simply folding over the remote ends of each of the wrapper sheets as is illustrated in Fig. 3. Alternatively, the tabs may be formed by leaving the remote ends free of adhesive, cohesive or heat sealable coatings.

In accordance with this invention, sealing means for adhering the wrapping sheets to the facing sheets are provided in the area of their face-to-face contact; these sealing means having the characteristic of providing greater resistance to peeling (peel strength) than the bond between the facing sheets and the folded bandage. As illustrated in Fig. 4, these sealing means comprise coating the wrapping sheets and the facing sheets in their area of face-to-face-contact with cohesive layers 34 and 36. It will be understood that layers 34 and 36 may be sealed by various alternative means now known in the art provided, of course, that the peel strength criteria set out herein are conformed to. Such alternative sealing means may be, for example, adhesive coatings, heat sealable coatings, or even such sealing methods as ultrasonic sealing or radio frequency sealing in the case where layers 34 and 36 are compatible materials for these sealing methods. In the preferred embodiment of Figs. 1—5, layers 30, 34 and 36 are all of the same material. While this specific embodiment is not absolutely essential, such embodiment greatly simplifies the manufacture of the packaged bandage of this invention. For example, by employing such embodiment, the wrapping sheets and the facing sheets may each be overall coated with the identical layer and there is no need for coating specific areas of the respective sheets with specific coating materials. Further, the need for precise registering of the faced folded bandage with respect to the wrapping sheets is obviated.

Fig. 2 illustrates the ease with which the user may open the packaged bandage of the invention. The two wrapping sheets 26 are gripped by the tabs 32 and pulled apart. Because of the sealing means between the wrapping sheets and the facing sheets 24 conform to the criteria set out herein, the same peeling action removes the facing sheets from the folded bandage and effectively unfolds the bandage and presents the same ready for use. The facing sheets remain adhered to the wrapping sheets and may be discarded therewith.

Referring now to Figs. 6—10, shown therein is a second embodiment of this invention. This second embodiment varies from that of Fig. 1—5, in that, unlike the two wrapping sheet packaged bandage of the first embodiment, this embodiment employs a single wrapping sheet, folded and fin-sealed about the bandage.

The packaged bandage 40 again comprises a folded bandage 42 consisting of a planar flexible backing 44 having a layer of pressure sensitive adhesive 46 coated on one surface and provided on that surface with a pad 48 for absorbing body exudate. The bandage is folded about fold line 50 to bring the backing into face-to-face relationship along interface 52 and present pressure sensitive adhesive 46 on each of its external major surfaces.

The folded bandage 42 is sandwiched between two facing sheets 54 to form a faced folded bandage.

A single, planar, generally rectangular wrapping sheet 56 is provided having parallel longitudinal edges 55 and parallel transverse edges 57 with the distance between known edges being somewhat longer than the longitudinal dimension of the folded bandage and the distance between the longitudinal edges being somewhat longer than twice the transverse dimension of the bandage.

The wrapping sheet 56 is wrapped around the faced folded bandage with the longitudinal edges 55 meeting above one major surface of the bandage and the transverse edge 57 extending beyond the transverse ends of the folded bandage. The portions of the wrapping sheet adjacent to the longitudinal edges 55 are sealed together and folded flat to form what is known in the packaging art as a "fin seal" 58. The portions of the wrapping sheet extending beyond the bandage are peelablely sealed together to completely enclose the bandage. The latter sealing and preferably the fin sealing may be accomplished using adhesive, cohesive, or heat sealable coatings on the appropriate portion of the wrapping sheet. Such coating is illustrated as layer 60 in Fig. 11. Tabs 62 are provided on at least one end of the package to facilitate peeling the wrapping seals. These tabs may be formed by slitting open a remote portion of the sleeve formed by the wrapping sheet 56 and folding the remote end of the slit portion onto itself, as is illustrated in Fig. 6—8.

In accordance with the present invention, sealing means for adhering the wrapping sheet to the facing sheets are provided in the area of their face-to-face contact and, as in the prior embodiment, such sealing means have the characteristic of providing greater resistance to peeling than that offered by the bond between the facing sheets and the folded bandage. The Fig. 10 illustrates such sealing means as layers 64 and 66 coated onto the facing sheets and wrapping sheet, respectively. As in the prior described embodiment, these layers may be of adhesive, cohesive, or heat sealable material. Once again it is preferred that layers 64, 66, and 60 all comprise a single material so that both the wrapping sheet and the facing sheets can be overall coated for ease in manufacturing the packaged bandage of this invention.

As is illustrated in Fig. 7, the wrapping sheet, gripped by the tabs 62 may be peeled away from the bandage and present the bandage ready to use. Because of the relatively stiff facing sheets the wrapping sheet will tear neatly along the longitudinal edges 68 of the facing sheets to expose the bandage. Because of the peel strength of the bond between the facing sheets and the wrapping sheet, as contrasted with the bond between the facing sheets and the bandage, the facing sheets will remain adhered to the wrapping sheet and can be disposed of therewith.

## Claims

1. A packaged pressure sensitive adhesive bandage wherein said bandage comprises a flexible, planar backing (14) having one surface thereof coated with a pressure sensitive adhesive (16); a pad (18) for absorbing body exudate located on the adhesively coated surface of the backing (14);

said bandage (12) being folded upon itself about a transverse fold line (20) to form a folded bandage with pressure sensitive adhesive coating (16) on each of its external major surfaces;

said folded bandage being sandwiched between facing sheets (24) to form a faced folded bandage, said facing sheets (24) adhering to said pressure sensitive adhesive (16);

said faced folded bandage being enclosed by a wrapper (26) with portions of the inside surfaces of said wrapper (26) overlying said facing sheets (24);

sealing means (34, 36) adhering said portions of the inside surface of said wrapper (26) to said facing sheets (24), said sealing means (34, 36) providing more tenacity between said portions and said facing sheets (24) than the tenacity of the adhesive (16) between said facing sheets (24) and said folded bandage (12);

whereby, when said package is opened by removing said wrapper (26), said facing sheets (24) will disengage from said folded bandage (12) and remain adhered to said wrapper (26), thereby presenting said bandage (12) ready for use.

2. The packaged bandage of claim 1 wherein said sealing means (34, 36) comprise an adhesive layer between said portions of said wrapper (26) and said facing sheets (24).

3. The packaged bandage of claim 1 wherein said sealing means comprise a cohesive layer (36) on said portions of said wrapper (26) cooperating with a cohesive layer (34) on said facing sheets (24) to adhere said facing sheets (24) to said wrapper (26).

4. The packaged bandage of claim 1 wherein said sealing means comprise a heat sealed coating between said portions of said wrapper (26) and said facing sheets (24).

5. The packaged bandage of any one of claims 1 to 4 wherein said wrapper comprises two panels (26) sandwiching said faced folded bandage (12) therebetween, said panels (26) having peripheral portions (28) extending beyond at least a portion of the periphery of said faced folded bandage (12), said peripheral portions (28) being sealed together to enclose said bandage (12).

6. The packaged bandage of claim 5 wherein said peripheral portions (28) are sealed together by a peelable seal.

7. The packaged bandage of any one of claims 1 to 4, wherein the wrapper comprises:

a rectangular wrapping sheet (56) having transverse edges (57) and longitudinal edges (55), the distance between said transverse edges (57) being longer than the longitudinal dimension of the folded bandage (42) and the distance between the longitudinal edges (55) being longer than twice the transverse dimension of the folded bandage (42), said wrapping sheet (56) being wrapped around the faced folded bandage (42) with the longitudinal edges (55) meeting above a major surface of the folded bandage (42) and the transverse edges extending beyond the transverse ends of the folded bandage (42);

the portions of said wrapping sheet (56) adjacent to said longitudinal edges being sealed together and folded flat to form a fin seal (58);

said portions of said wrapping sheet (56) adjacent the transverse edges (57) being peelably sealed together to enclose said folded bandage (42).

8. The packaged bandage of any one of claims 5 to 7, wherein said peripheral portions (28) extend beyond at least one transverse edge of said folded bandage (12) and are provided with tabs (32) for peeling said seal apart.

9. The packaged bandage of any one of claims 5 to 8, wherein said peripheral portions (28) are sealed with a heat sealed layer therebetween.

## Patentansprüche

1. Verpacktes, selbsthaftendes Heftpflaster, wobei das genannte Pflaster eine biegsame, ebene Unterlage (14), die auf einer ihrer Oberflächen mit einem Haftkleber (16) beschichtet ist, sowie

ein Kissen (18) zum Absorbieren von Körperexsudat, welches Kissen auf der mit dem Haftkleber beschichteten Oberfläche der Unterlage (14) angeordnet ist, aufweist;

und wobei das genannte Pflaster (12) unter Bildung eines gefalteten Pflasters, welches auf jeder seiner Hauptaußenseiten mit einem Haftkleberüberzug (16) versehen ist, um eine Querfaltungslinie (20) auf sich selbst gefaltet ist;

und wobei das genannte gefaltete Pflaster unter Bildung eines mit einer Abdeckung versehenen, gefalteten Pflasters zwischen Abdeckblätter (24) eingelegt ist, welche Abdeckblätter (24) an dem genannten Haftkleber (16) anhaften;

und wobei das genannte, mit einer Abdeckung versehene gefaltete Pflaster von einer Umhüllung (26) umschlossen ist, wobei Teile der Innenseiten der genannten Umhüllung (26) über den genannten Abdeckblättern (24) zu liegen kommen;

und wobei das genannte Pflaster weiterhin Versiegelungsmittel (34, 36) aufweist, mittels welcher die genannten Teile der Innenseite der genannten Umhüllung (26) an den genannten Abdeckblättern (24) anhaften; und wobei die genannten Versiegelungsmittel (34, 36) eine größere Klebrigkeit zwischen den genannten Teilen und den genannten Abdeckblättern (24) schaffen als die Klebrigkeit des Klebers (16) zwischen den genannten Abdeckblättern (24) und dem genannten gefalteten Pflaster (12) ausmacht;

wodurch dann, wenn die genannten Verpackung durch Entfernen der genannten Umhüllung (26) geöffnet wird, sich die genannten Abdeckblätter (24) von dem genannten gefalteten Pflaster (12) ablösen und an der genannten Umhüllung (26) anhaften bleiben werden, wodurch sich das genannte Pflaster (12) gebrauchsfertig darbietet.

2. Verpacktes Heftpflaster nach Anspruch 1, wobei die genannten Versiegelungsmittel (34, 36) eine Kleberschicht zwischen den genannten Teilen der genannten Umhüllung (26) und den genannten Abdeckblättern (24) umfassen.

3. Verpacktes Heftpflaster nach Anspruch 1, wobei die genannten Versiegelungsmittel eine Kohäsionsschicht (36) auf den genannten Teilen der genannten Umhüllung (26) umfassen, welche Kohäsionsschicht (36) mit einer Kohäsionsschicht (34) auf den genannten Abdeckblättern (24) derart zusammenwirkt, daß dadurch die genannten Abdeckblätter (24) mit der genannten Umhüllung (26) zusammengeklebt werden.

4. Verpacktes Heftpflaster nach Anspruch 1, wobei die genannten Versiegelungsmittel einen heißgesiegelten Überzug zwischen den genannten Teilen der genannten Umhüllung (26) und den genannten Abdeckblättern (24) umfassen.

5. Verpacktes Heftpflaster nach einem der Ansprüche 1 bis 4, wobei die genannte Umhüllung zwei Blätter (26) umfäßt, zwischen welchen das genannte, mit einer Abdeckung versehene gefaltete Pflaster (12) eingelegt ist, wobei die genannten Blätter (26) Randteile (28) aufweisen, die sich über wenigstens einen Teil des Randes des genannten, mit einer Abdeckung versehenen gefalteten Pflasters (12) hinaus erstrecken, und wobei die genannten Randteile (28) miteinander derart versiegelt sind, daß sie das genannte Pflaster (12) zwischen sich einschließen.

6. Verpacktes Heftpflaster nach Anspruch 5, wobei die genannten Randteile (28) miteinander durch eine abziehbare Versiegelung versiegelt sind.

7. Verpacktes Heftpflaster nach einem der Ansprüche 1 bis 4, wobei die Umhüllung umfaßt: ein rechteckiges Umhüllungsblatt (56) mit Querkanten (57) und Längskanten (55), wobei der Abstand zwischen den genannten Querkanten (57) länger als die Abmessung des gefalteten Pflasters (42) in der Längsrichtung ist, und wobei der Abstand zwischen den Längskanten (55) länger als die zweifache Abmessung des gefalteten Pflasters (42) in der Querrichtung ist, und wobei das genannte Umhüllungsblatt (56) um das mit einer Abdeckung versehene, gefaltete Pflaster (42) derart herumgehüllt ist, daß die Längskanten (55) oberhalb einer Hauptoberfläche des gefalteten Pflasters (42) aufeinandertreffen, und daß sich die Querkanten über die in der Querrichtung verlaufenden Enden des gefalteten Pflasters (42) hinaus erstrecken;

und wobei die neben den genannten Längskanten befindlichen Teile des genannten Umhüllungsblattes (56) unter Bildung einer Nahtversiegelung (58) miteinander versiegelt und flach gefaltet sind;

und wobei die neben den Querkanten (57) befindlichen Teile des genannten Umhüllungsblattes (56) unter Einschluß des genannten gefalteten Pflasters (42) abziehbar miteinander versiegelt sind.

8. Verpacktes Heftpflaster nach einem der Ansprüche 5 bis 7, wobei sich die genannten Randteile (28) über wenigstens eine Querkante des genannten gefalteten Pflasters (12) hinaus erstrecken und mit Laschen (32) ausgestattet sind, welche dazu dienen, die genannte Versiegelung auseinanderzuziehen.

9. Verpacktes Heftpflaster nach einem der Ansprüche 5 bis 8, wobei die genannten Randteile (28) mit einer dazwischen befindlichen, heißgesiegelten Schicht versiegelt sind.

**Revendications**

1. Pansement adhésif emballé ahérant par pression, dans lequel ledit pansement comprend un support plan flexible (14) dont une surface est enduite d'un adhésif adhérant par pression (16); un tampon (18) pour absorber les exudats physiologiques disposé sur la surface enduite d'adhésif du support (14); ledit pansement (12) étant plié sur lui-même suivant une ligne transversale de pliage (20) pour former un pansement plié portant en enduit d'adhésif adhérant par pression (16) sur chacune de ses surfaces extérieures principales; ledit pansement plié étant en sandwich entre des feuilles extérieures (24) pour former un pansement plié recouvert, lesdites feuilles extérieures (24) adhérant audit adhésif qui adhère par pression (16); ledit pansement plié recouvert étant entouré d'une enveloppe (26), des portions des surfaces intérieures de ladite enveloppe (26) recouvrant lesdites feuilles extérieures (24); des moyens de scellement (34, 36) faisant adhérer lesdites portions de la surface intérieure de l'enveloppe (26) auxdites feuilles extérieures (24), lesdits moyens de scellement (34, 36) assurant plus de tenacité entre lesdites portions et lesdites feuilles extérieures (24) que la ténacité de l'adhésif (16) entre les feuilles extérieures (24) et ledit pansement plié (12); de sorte que, quand on ouvre l'emballage en enlevant ladite enveloppe (26), lesdites feuilles extérieures (24) se détachent dudit pansement plié (12) et restent collées à ladite enveloppe (26) pour présenter ainsi ledit pansement (12) prêt à servir.

2. Pansement emballé selon la revendication 1, dans lequel lesdits moyens de scellement (34, 36) comprennent une couche adhésive entre lesdites portions de ladite enveloppe (26) et lesdites feuilles extérieures (24).

3. Pansement emballé selon la revendication 1, dans lequel lesdits moyens de scellement comprennent une couche cohésive (36) sur lesdites portions de ladite enveloppe (26) qui coopère avec une couche cohésive (34) sur lesdites feuilles extérieures (24) pour faire adhérer lesdites feuilles extérieures (24) à ladite enveloppe (26).

4. Pansement emballé selon la revendication 1, dans lequel lesdits moyens de scellement comprennent un enduit thermoscellé entre lesdites portions de ladite enveloppe (26) et lesdites feuilles extérieures (24).

5. Pansement emballé selon l'une quelconque des revendications 1 à 4, dans lequel ladite enveloppe comprend deux panneaux (26) qui mettent en sandwich ledit pansement plié recouvert (12) entre eux, lesdits panneaux (26) ayant des portions périphériques (28) s'étendant au delà d'au moins une portion de la périphérie du pansement plié recouvert (12), lesdites parties périphériques (28) étant scellées ensembles pour entourer ledit pansement (12).

6. Pansement emballé selon la revendication 5, dans lequel lesdites parties périphériques (28) sont scellées ensemble par un joint amovible.

7. Pansement emballé selon l'une quelconque des revendications 1 à 4, dans lequel l'enveloppe comprend: une feuille rectangulaire d'emballage (56) ayant des bords transversaux (57) et des bords longitudinaux (55), la distance entre lesdits bords transversaux (57) étant plus longue que la dimension longitudinale du pansement plié (42) et la distance entre les bords longitudinaux (55) étant plus longue que le double de la dimension transversale du pansement plié (42), ladite feuille d'enveloppe (56) étant placée autour du pansement plié recouvert (42), les bords longitudinaux (55) se rejoignant au dessus d'une surface principale du pansement plié (42) et les bords transversaux s'étendant au delà des extrémités transversales du pansement plié (42); les parties de la feuille d'emballage (56) adjacentes auxdits bords longitudinaux étant scellées ensemble et pliées à plat pour former un joint à ailettes (58); lesdites portions de la dite feuille d'emballage (56) à côté des bords transversaux (57) étant scellées ensemble de façon amovible pour enfermer ledit pansement plié (42).

8. Bandage emballé selon l'une quelconque des revendications 5 à 7, dans lequel lesdites portions périphériques (28) s'étendent au delà d'au moins un bord transversal dudit pansement plié (12) et sont munies de languettes (32) pour ouvrir ledit scellement.

9. Pansement emballé selon l'une quelconque des revendications 5 à 8, dans lequel lesdites portions périphériques (28) sont scellées avec interposition d'une couche thermoscellée.

FIG.1.

FIG.2.

FIG.4.

FIG.5.

FIG.3.

0 101 298

FIG.6.

FIG.10.

FIG.9.

FIG.7.

FIG.11.

FIG.8.

0 101 298